# EUROPEAN PATENT SPECIFICATION

(11) **EP 2 294 984 B1**
(45) Date of publication and mention of the grant of the patent: **12.10.2016**
(21) Application number: 10012252.2
(22) Date of filing: 17.06.2004
(51) Int. Cl.: A61B 10/00, A61B 17/28, A61B 17/29, A61B 10/06, A61B 17/00, A61B 17/32

(54) **Endoscopic instrument**
Endoskopisches Instrument
Instrument endoscopique

(30) Priority: 18.06.2003 US 479145 P; 17.02.2004 US 778226; 14.05.2004 US 845108
(43) Date of publication of application: 16.03.2011
(62) Divisional of application: 04753450.8
(73) Proprietor: Boston Scientific Limited, Hamilton HM11 (BM)
(72) Inventor: Rothberg, Elliott, Coral Spring, FL 33076 (US); Gil de Montes, William, Pembroke Pines, FL33029 (US); Zardeskas, James M., Pascoag, RI02859 (US); Freed, David I., Westborough, MA 01581 (US); Magill, Michael J., Northboro, MA 01532 (US); Sharma, Satish, Randolph, MA 02368 (US); Gingrich, Jon, Lancaster, PA17601 (US); Boarini, Edward, Holliston, MA 01746 (US)
(74) Representative: Vossius & Partner Patentanwälte Rechtsanwälte mbB

(56) References cited:
- WO-A1-96/09004
- JP-A- 2002 034 989
- JP-A- 2002 153 475

## Description

### Field of the Invention

Embodiments of the disclosure include a medical device with one or more of a variety of features. More particularly, embodiments of the disclosure relate to endoscopic devices that include one or more features that improve the use of the device. Examples of such features include chamfered edges and corners on, for example, the end effectors, a surface with a controlled finish also on, for example, the end effectors, a jaw with teeth and/or a tang having various configurations, a handle having soft-grip features, and/or an elongate member with varied rigidity. Other examples of such features include a folded portion on, for example, the end effectors and/or a snap-fit clevis assembly.

### Background of the Invention

Various medical instruments may be used in connection with an endoscope for performing a number of operations at a site deep within a patient's body cavity. One such instrument, a biopsy forceps device, samples tissue from a body cavity with minimal intervention and discomfort to patients. Typically, a biopsy forceps device, like other endoscopic instruments, has a long flexible tubular member for insertion into a lumen of an endoscope. The tubular member is sufficiently long and flexible to follow a long, winding path of the body cavity. An end effector assembly, such as a biopsy forceps assembly, is attached at a distal end of the tubular member, and a handle is attached at a proximal end of the tubular member. The handle may have an elongate portion and a spool portion disposed around the elongate portion. The spool portion may be configured to move longitudinally relative to the elongate portion. An elongate mechanism, such as one or more pull wires, extends through the tubular member to connect the end effector assembly and a portion of the handle, such as the spool portion. Longitudinal movement of the spool portion relative to the elongate portion of the handle causes the elongate mechanism to move longitudinally in the tubular member, which in turn causes the actuation of the end effector assembly.

In methods of using the biopsy forceps device, an endoscope is placed in a patient's body cavity adjacent a tissue site from which the acquisition of a tissue sample is desired. The biopsy forceps device is then advanced to the tissue site via a working channel of the endoscope. Once the biopsy forceps device is next to the portion of the tissue from which the acquisition of a tissue sample is desired, the spool portion is moved relative to the elongate portion so as to move pull wires. The movement of the pull wires causes the jaws of the biopsy forceps assembly to open. The open jaws are then advanced to the tissue site, and the spool portion is again moved relative to the elongate portion so as to move the pull wires such that the jaws close. The closing of the jaws causes a tissue sample to be captured in the end effector assembly. The biopsy forceps device is then removed from the body cavity via the working channel of the endoscope.

### SUMMARY OF THE INVENTION

The invention includes a medical device as defined in claim 1. Further embodiments of the invention are defined in the dependent claims.

Document JP 2002-034989 A discloses a forceps cup wherein a metal plate was folded over.

### BRIEF DESCRIPTION OF THE DRAWINGS

The accompanying drawings, which are incorporated in and constitute a part of this specification, illustrate embodiments and together with the description, serve to explain the principles of the invention.
Fig. 1 is a perspective view of an endoscopic instrument suitable for use in connection with embodiments of the present disclosure.
Fig. 2 is a perspective view of a jaw portion of an endoscopic instrument.
Fig. 3 is a perspective view of a jaw portion of an endoscopic instrument according to an embodiment of the present disclosure.
Fig. 4 is a schematic view of an endoscopic instrument with an elongate member of variable flexibility according to an embodiment of the present disclosure.
Fig. 5 is a perspective view of a jaw assembly of an endoscopic instrument according to an embodiment of the present disclosure.
Fig. 6 is a perspective view of a jaw assembly of an endoscopic instrument according to an embodiment of the present disclosure.
Fig. 7 is a view of a jaw portion of the jaw assembly of Fig. 6.
Fig. 8A is a side view of mated jaw portions of an endoscopic instrument.
Fig. 8B is a side view of mated jaw portions of an endoscopic instrument.
Fig. 9 is a side view of a jaw portion of the jaw assembly of Fig. 6.
Fig. 10 is a side view of the mated jaw portions of Fig. 9.
Fig. 11 is a side view of a jaw portion of an endoscopic instrument according to another embodiment of the present disclosure.
Fig. 12 is a side view of a jaw portion of an endoscopic instrument according to yet another embodiment of the present disclosure.
Fig. 13 is a top view of a tang portion and control wire of an endoscopic instrument.
Fig. 14 is a top view of a tang portion and control wire of an endoscopic instrument according to an embodiment of the present disclosure.
Fig. 15A is a side view of a jaw with a tang portion, having an unfolded additional section, of an endoscopic instrument according to the present invention.
Fig. 15B is a perspective view of the jaw with the tang portion of Fig. 15A, with the additional section folded, according to the present invention.
Fig. 16 is a side view of a handle of an endoscopic instrument according to an embodiment of the present disclosure.
Fig. 17 is a side view of a handle of an endoscopic instrument according to another embodiment of the present disclosure.
Fig. 18A is a side view of a tang portion of a jaw according to a further embodiment of the present disclosure.
Fig. 18B is a cross-sectional view of the tang portion of Fig. 18A along line 18B-18B.
Fig. 18C is a cross-sectional view of a tang portion of a jaw according a still further embodiment of the present disclosure.
Fig. 18D is a cross-sectional view of a tang portion of a jaw according a yet further embodiment of the present disclosure.
Fig. 19A is a perspective view of a clevis assembly according to yet another embodiment of the present disclosure.
Fig. 19B is a side view of an axle of the clevis assembly of Fig. 19A.
Fig. 19C is a partial side view of a portion of the clevis assembly of Fig. 19A.
Fig. 19D is a schematic view of the clevis assembly of Fig. 19A.
Fig. 19E is a schematic view of the clevis assembly of Fig. 19A, with the axle being inserted into the clevis.
Fig. 20A is a side view of a clevis according to still another embodiment of the present disclosure.
Fig. 20B is a schematic view of an axle in the clevis of Fig. 20A.
Fig. 20C is a schematic view of the axle and clevis of Fig. 20A.

### DESCRIPTION OF THE EMBODIMENTS

Reference will now be made in detail to the present exemplary embodiments illustrated in the accompanying drawings. Wherever possible, the same reference numbers will be used throughout the drawings to refer to the same or like parts.

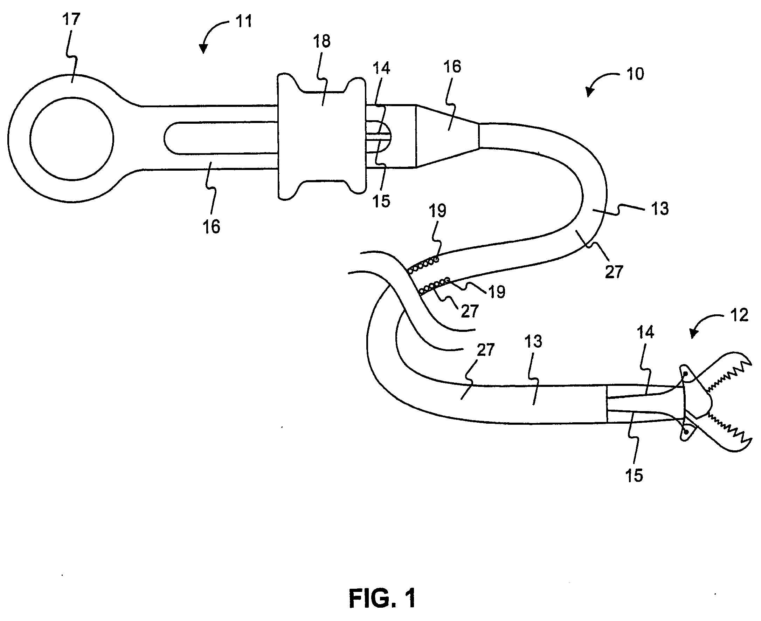

An exemplary embodiment of a medical device is depicted in Fig. 1. The medical device is an endoscopic instrument 10 that includes a handle portion 11 and an end effector assembly 12 connected to each other by a flexible elongate member 13. Control wires 14, 15 extend between the handle portion 11 and the end effector assembly 12 via a lumen in the flexible elongate member 13. The handle portion 11 includes an elongate portion 16 connected at its proximal end to a ring portion 17 and a spool portion 18 slidably disposed around the elongate portion 16. The elongate member 13 may having a coiled portion 19 (partially shown in Fig. 1) covered by an outer jacket or a sheath 27. However, the elongate member 13 may not have a coiled portion 19, and instead may include a single layer tubular member. The end effector assembly 12 may be any type of assembly, for example, a biopsy forceps jaw as depicted in Fig. 1. The control wires 14, 15 may be connected at their distal ends to opposing portions of the end effector assembly 12, and at their proximal ends to spool portion 18. Longitudinal movement of the spool portion 18 relative to the elongate portion 16 causes the actuation of the end effector assembly 12 via the control wires 14, 15. Portions of the control wires 14, 15 disposed in the handle 16 may be contained within a tube also disposed in the handle 16. The tube may provide the compressive strength that may be needed to actuate the end effector assembly 12.

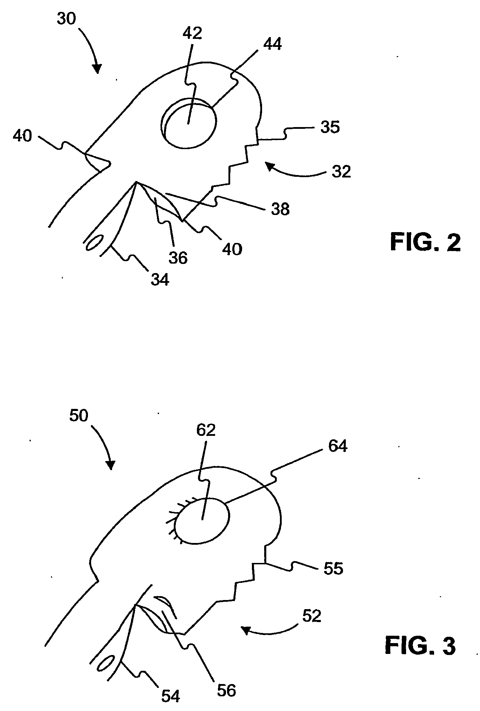

A current biopsy forceps jaw 30, such as that shown in Fig. 2, includes a jaw 32 extending from an arm 34. Jaw 32 includes a sharp edge or teeth 35 at its cutting edge. Teeth 35 may mate with another biopsy forceps jaw, of like or similar construction, of an endoscopic forceps instrument to obtain a biopsy sample. Jaw 32 also includes flat surfaces on various parts of jaw 32. For example, the back or proximal-most surface 36 of jaw 32 and certain surfaces intersecting with surface 36 may be flat. The intersection of those surfaces will result in sharp comers and edges, such as edges 38 and corners 40. Jaw 32 also defines a fenestration hole 42 that may include a sharp edge 44. Many current biopsy forceps jaws have such a construction because they are cast from a molded plastic pattern. Certain efficiencies in the manufacture of injection molds lead to flat, intersecting planes and sharp edges and corners of the resultant jaws. These sharp edges and corners, however, may get caught within an endoscope working channel upon entry or exit of a biopsy forceps device through that channel or at the distal end of the endoscope upon re-entry of the forceps after use.

Embodiments include a medical device or portions of the medical device with chamfered corners and/or edges. Fig. 3 shows a biopsy forceps jaw 50 according to an exemplary embodiment.
The biopsy forceps jaw 50 includes a jaw 52 extending from an arm 54. Like jaw 32 of Fig. 2, jaw 52 includes a sharp edge or teeth 55 at a cutting edge. Unlike jaw 32, however, certain surfaces of jaw 52 are not substantially flat and, instead, are rounded at least near the edges of those surfaces. The corners and edges of various intersecting surfaces are therefore chamfered, beveled, rounded, and/or radiused off and not sharp. For example, the back or proximal-most surface 56 of jaw 52 and certain surfaces intersecting with surface 56 are rounded at least near the edges of those surfaces so that there are no, or fewer, sharp edges or corners associated with jaw 32 (other than the sharp cutting edge having teeth). Jaw 52 also defines a fenestration hole 62 that may include an edge 64 that is rounded, chamfered, beveled, and/or radiused, off so that there is not a sharp edge. The resulting jaw will have no, or fewer, sharp edges or corners to catch within an endoscope working channel upon entry or exit of a biopsy forceps device through that channel or at the distal end of the endoscope upon re-entry of the forceps after use. Less interference with at least the distal section of the endoscope results.

Providing a medical device, or portions thereof, with non-sharp edges and corners may apply to other types of end effectors or other parts of endoscopic or non-endoscopic instruments, including, but not limited to graspers, scissors, forceps, or other laproscopic, endoscopic, or other devices. For example, the medical device may have a sharp cutting edge that is a radial edge (i.e., a straight cutting edge with no teeth). Other edges, corners, and surface intersections, aside from those mentioned above, may be rounded, chamfered, beveled, and/or radiused off as desired to minimize the effects associated with sharp regions as the device is being used. For example, other portions of the end effector assembly, including tang portions, clevis portions, and/or axle portions may include rounded, chamfered, beveled, and/or radiused off edges and corners.

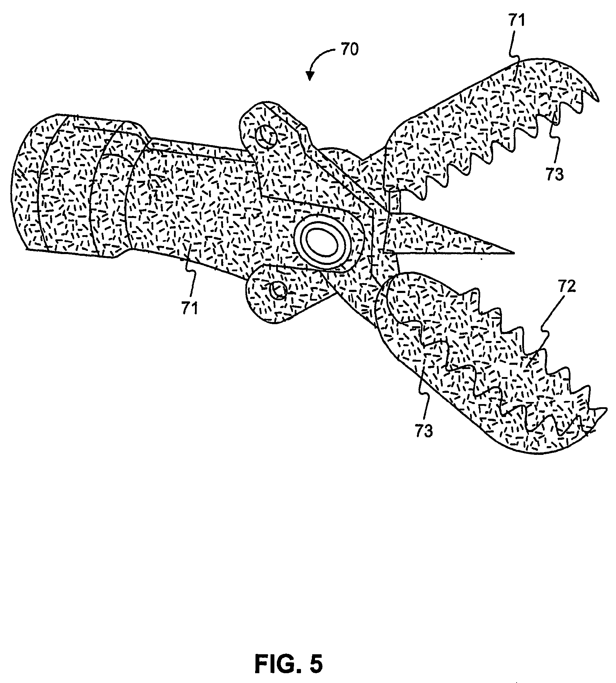

Embodiments include a medical device or portions of the medical device having a controlled surface finish, including a roughened surface finish. Fig. 5 shows the inner surface 72 and outer surface 71 of a biopsy forceps jaw assembly 70 having a rough surface finish. While Fig. 5 shows a biopsy forceps jaw assembly 70 having all parts with a roughened surface, less than all of the parts of the jaw assembly 70 may include a roughened or textured surface. For example, to attain many of the advantaged described herein, it may be desirable for only the jaws 73, or portions of the jaws 73 such as the outer surface 71, to have a roughened or otherwise textured surface.

Tissues are less prone to sticking to surfaces of jaws having a rough finish than surfaces of jaws having a smooth finish. For example, tissue samples cut with the roughened jaws 73 may be less prone to sticking to the surfaces 71, 72 of the jaws 73. By lessening the smoothness of the inner surface 72 of the jaws 73, the tissue sample may be more easily removed from the jaws 73, for example, when the tissue sample is discharged into an external container.

One potential advantage of having a controlled roughness on the surface of the jaws is that by reducing the amount of sticking, surface contact, and/or seal between the tissue samples and the biopsy jaws, the amount of time spent in a biopsy tissue acquisition procedure is reduced. For example, the amount of time spent trying to release the surface contact between the tissue samples and the surfaces of the jaws, during multiple sample acquisition and/or removing the samples from the jaws into an external specimen container, is reduced. This may permit faster turnaround when a single bite biopsy forceps assembly needs to be removed from an endoscope, the tissue sample retrieved from the jaw, and the assembly reinserted into the endoscope to obtain a subsequent sample.

Another potential advantage for having a rough finish on the surface of the endoscopic instrument is that it reduces surface contact between jaws and/or prevents surfaces of the jaws from sealing and/or sticking to each other. Smooth surfaces may sometimes stick together and form a seal, particularly if a fluid is placed between the surfaces. Having a rough finish on the surface of the jaws reduces the force with which that particular surface of the jaws will stick to either each other or another surface. For example, the surfaces of the teeth of opposing jaws may be less prone to sticking to each other when brought together.

Yet another potential advantage for controlling the surface finish of an endoscopic instrument is that it may provide a more consistent feel and/or performance to the user. For example, the entire endoscopic instrument may have a particular finish, or portions of the endoscopic instrument, such as the end effectors, may have different finishes.

A further potential advantage for controlling the surface finish of an endoscopic instrument is that, for example, when an optimum level of roughness is provided to the surface of the jaw assembly, tissue is more readily grasped and retained in the jaws, for example, so that multiple samples may be collected with a single bite forceps. The controlled surface texture may allow a user to obtain subsequent tissue samples with the prior sample(s) remaining within the jaws. A particular texture of the jaws may allow the tissue sample to be retained within the open jaws while the user acquires a second sample.

A still further potential advantage for controlling the surface finish of an endoscopic instrument is that, for example, when an optimum level of roughness is provided to the surface of the jaw assembly, the roughened surface may assist in both retaining and removing the sample. Such assistance may be dependent on the presence or absence of an external force. For example, when there is no external force exerted on the sample, the roughened surface may assist in the retention of the sample. In another example, when an external force is applied to the sample, the roughened surface may assist in the removal of the sample.

The roughness of the surfaces 71, 72 of the jaw assembly 70 may be created and/or adjusted, for example, by controlling the casting of the jaws 73 and/or subsequent processing of the jaw assembly 70. Subsequent processing may including grit blasting, media tumbling, and/or any other suitable surface finishing technique. The surfaces 71, 72 of the jaw assembly 70 could also be plated, sputtered, photo-etched, acid-etched, and/or plasma coated to control the roughness of the surface. The surface or surfaces of the endoscopic instrument may have a roughness in the range of a few hundred microinches, and may be varied, for example, by increments of a few hundred microinches. The relative roughness of the surface or surfaces of the endoscopic instrument may be varied with respect to each other. For example, one surface or portion of a surface may have a relatively rough finish, while another surface or portion of a surface may have a relatively smooth finish.

Providing surface(s) of a medical device, or portions thereof, with a controlled finish, for example a roughened surface, may apply to other types of end effectors or other parts of endoscopic or non-endoscopic instruments, including, but not limited to graspers, scissors, forceps, or other laproscopic, endoscopic, or other devices. Furthermore, other portions of the end effector assembly, including tang portions, clevis portions, and/or axle portions may include surfaces with a controlled finish, for example, a roughened surface. Additionally, only specific portions of parts of the end effector assembly may have a controlled finish. For example, only the inner surfaces of a the jaws of an end effector assembly may have a roughened surface.

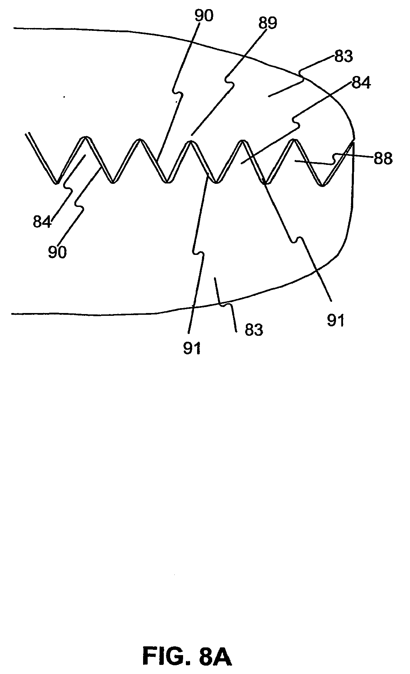

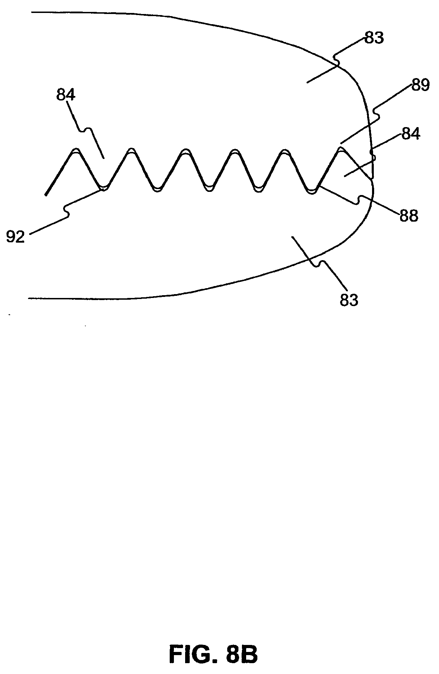

Views of mated jaw portions 83 of endoscopic instruments are shown in Figs. 8A and 8B. Each jaw portion 83 has teeth 84, with each tooth 84 having a crest portion 88. A root portion 89 is disposed between each set of adjoining teeth 84. Substantially diagonal portions 90 of the teeth 84 are disposed between the crest 88 and the root 89 to form the tooth.

The configuration of the root 89 may limit the configuration of the teeth. For example, in order for opposing teeth 84 to fit together, the substantially diagonal portions 90 of teeth 84 on opposing jaw portions 83 need to meet before the crest 88 contacts the root 89. Otherwise, a gap 91 will form between the substantially diagonal portions 90 of opposing jaw portions 83, as shown in Fig. 8A. The gap 91 may prevent the opposing jaw portions 83 and teeth 84 from performing an effective cutting action. Though Fig. 8A includes jaws 83 having teeth 84 with sharp tips to enhance biting action, it may be difficult to fabricate jaws (such as through stamping) that have matching sharp-comered roots 89.

In some cases, to ensure the opposing jaws portions 83 fully close, as shown in Fig. 8B, the crest portion 88 may be given a radius (about 0.005 inches) slightly larger than the radius of the root portion 89 (such as about 0.003 inches). A gap 92 is formed between the crest portion 88 of one jaw portion 83 and the root portion 89 of an opposing jaw portion 83. However, this jaw configuration includes teeth with non-sharp tips (i.e. crests) inhibiting optimal cutting performance.

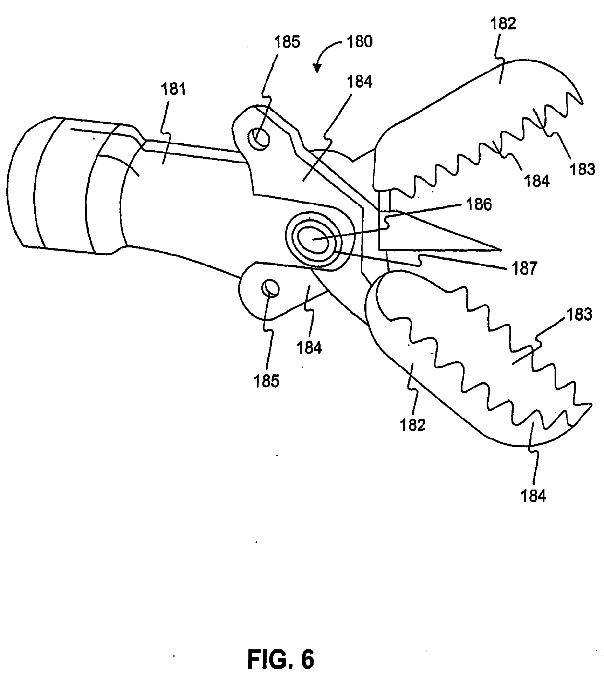

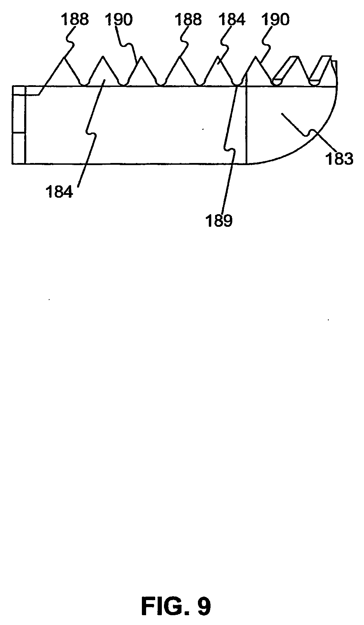

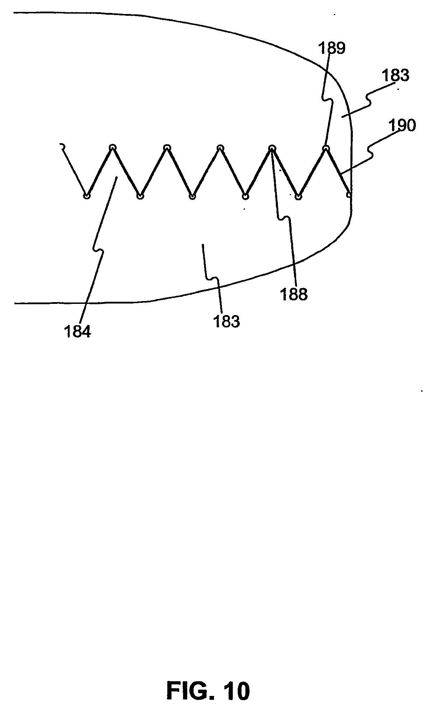

Embodiments include a medical device having jaws with various tooth and/or teeth configurations that overcome one or more of the drawbacks. A jaw assembly 180 according to an exemplary embodiment is depicted in Figs. 6, 7, and 9. The jaw assembly 180 includes a clevis 181 configured to be connected to the end of an elongate member 13. Opposing jaws 182 are rotatably attached to the distal end of the clevis 181. Each jaw 182 has a jaw portion 183 connected to a tang portion 184 with mounting holes 185 on the proximal end of the tang portion 184. The holes 185 may be configured to receive and/or retain a wire 15 or other interface device via the clevis 181. Each tang portion 184 also has an axle hole 186 configured to receive an axle 187 that may be connected to the clevis 181. Each jaw portion 183 has a plurality of teeth 184 configured to mate with the plurality of teeth 184 disposed on an opposing jaw portion 183. Material may be removed from the root 189 of adjoining teeth 184 so that, for example, sharper teeth (i.e., crest portions with smaller or no radii) may be used. As shown in Fig. 9, the root 189 has a circular cutout below the point where the crest 188 of an opposing jaw portion 183 would be captured, regardless of the sharpness of the crest 188 (i.e., the crest 188 may have a substantially zero radius). An example of such a configuration is depicted in Fig. 10. Accordingly, the crest 188 may be as sharp as desired, while still allowing the substantially diagonal portions 190 of opposing jaw portions 183 to come into contact with each other. Methods of sharpening teeth 184 such that the crest 188 has a substantially zero radius are known in the art (e.g., stamping, filing, casting). This jaw portion 183 configuration is advantageous as a sharper crest 188 results in a sharper tooth with an improved bite performance.

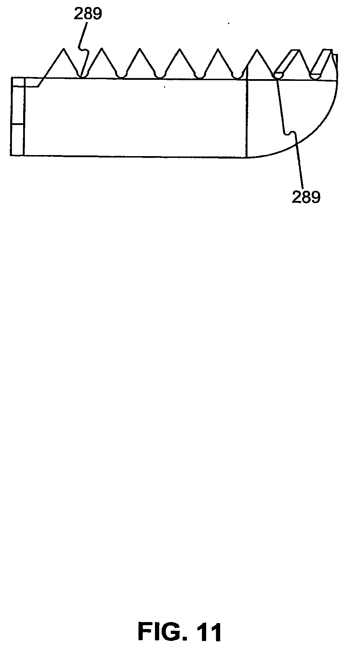

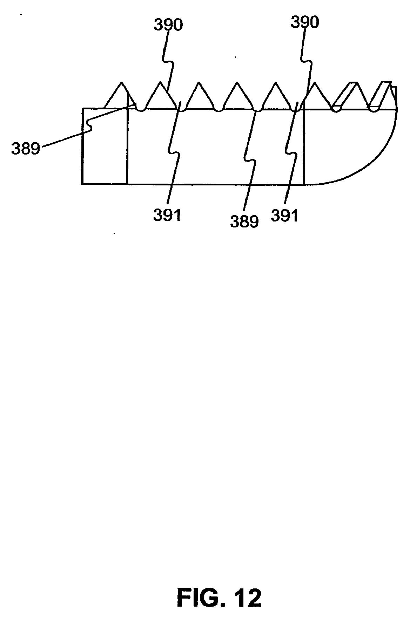

In various embodiments, the cutout portions of the root may have any shape or configuration that permits contact between substantially diagonal portions of opposing jaws that include sharp teeth. For example, Fig. 11 shows a root 289 configuration where the cutout is substantially U-shaped. In another example, Fig. 12 shows a root 389 configuration where the circular cutout is shifted vertically. Each root 389 has a center 391 that is disposed below the lower end of the substantially diagonal surfaces 390. In yet another example, the root portion and/or the circular cutout may also be shifted horizontally, so long as the substantially diagonal portions of the opposing jaw portions come into contact with each other without crests contacting the corresponding roots. In various embodiments, there may be a gap between the tip of the crest and the root, however, the tip of the crest may also just touch the lowest point of the root.

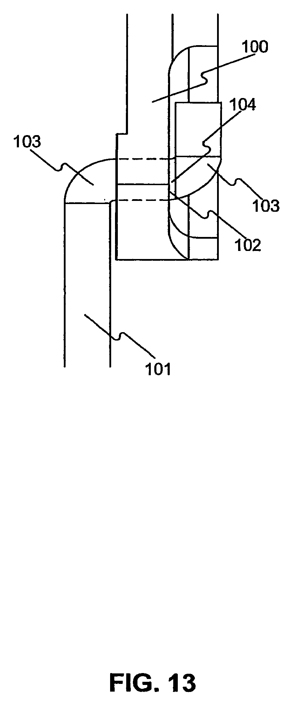

Fig. 13 shows a profile of a tang portion 100 of an end effector assembly for a medical instrument, with a wire 101 disposed in a mounting hole 102 of the tang portion 100. The end portion of the wire 101 has a roughly Z-shaped configuration so as to lodge the wire 101 in the hole 102, allow the wire 101 to rotate with respect to the hole 102, and/or prevent the wire 101 from falling out of the hole 102. The wire end portion has two bends 103 with an interface portion 104 between the bends 103 that contacts the internal surface of the hole 100. The interface portion 104 has substantially the same length as the axial length of the hole 102 and/or the width of the tang 100, for example, to prevent the wire 101 from shifting in the hole 102 and/or falling out of the hole 102. Two methods of forming the roughly Z-shaped configuration (i.e., bends 103) include stamping and/or forging a straight wire 101 into the roughly Z-shaped configuration, however, any method known in the art may be used. If the Z-shape is formed by a stamping or forging operation, the minimum length of the interface portion 104 (i.e., the portion of the wire between the bends) that may be formed is about 0.381 mm (0.015 inches).

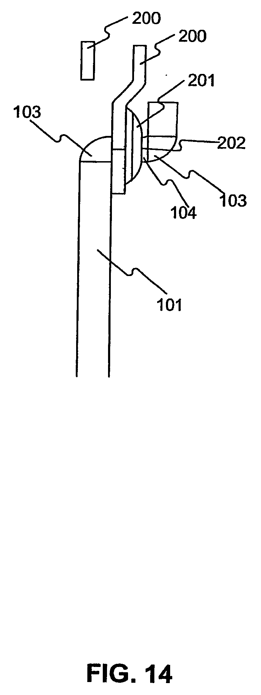

Embodiments of the invention include a medical device having an end effector assembly with various tang configurations. In an exemplary embodiment, a substantially narrow tang portion may have a widened portion, for example, by placing a dimple 201 on a tang portion 200 around a mounting hole 202. For example, as shown in Fig. 14, the dimple 201 may extend from the surface of the tang portion 200 and increase the width of the tang portion 200. The dimple 201 may be stamped onto the tang portion 200 so as to increase the width of the tang portion 200. This is advantageous because it allows the tang portion 200 and/or the rest of the jaw assembly to have a smaller thickness while still allowing the jaw assembly to accommodate the end portion of the wire 101 set forth above. Specifically, it allows the thickness of the tang portion 200 without the dimple 201 to be reduced, while still allowing the tang portion 200 and/or the mounting hole 202 to receive and accommodate an end portion of a wire 101 with an interface portion 104 having a length of about 0.381 mm (0.015 inches). For example, if the width of the tang portion 200 is about 0.1778 mm (0.007 inches), a dimple 201 of about 0.2032 mm (0.008 inches) could be added to the tang portion 200 so as to accommodate an end portion of a wire 101 with an interface portion 104 having a length of about 0.381 mm (0.015 inches), without the end portion of the wire 101 undesirably shifting in and/or falling out of the mounting hole 202. This is especially advantageous when manufacturing a stamped jaw (with tang) having a thickness of material that is less than the length of the interface portion 104.

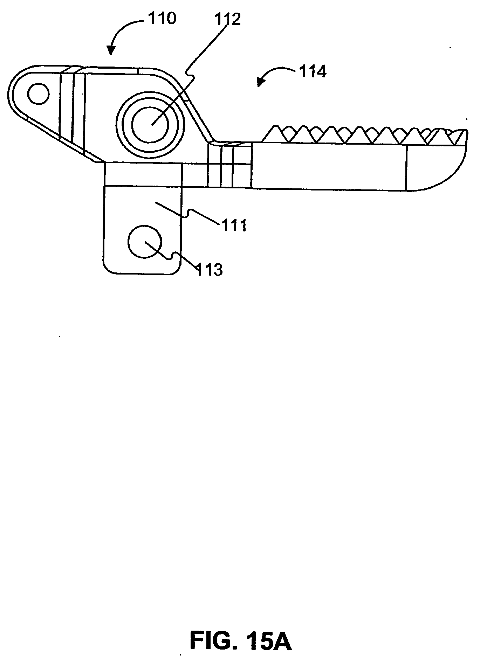

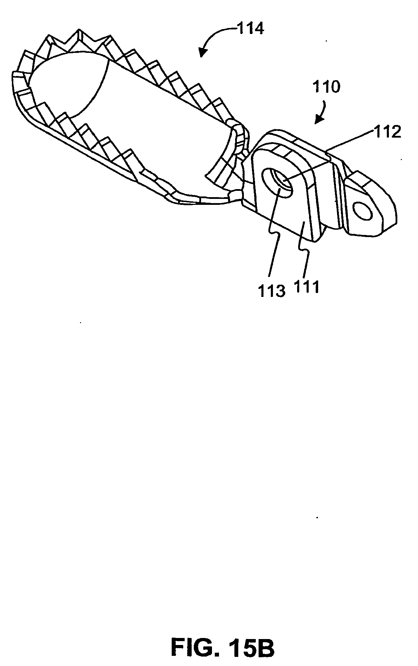

In various embodiments, the bends 103 need not make the end portion of the wire 101 into necessarily a roughly Z-shaped configuration. For example, the bends 103 could form the end portion of the wire 101 into a roughly U-shaped configuration. In addition, the bends 103 may be formed using any method known in the art. Furthermore, the dimples 201 may be formed using any method known in the art. For example, material may be soldered on and/or attached to the tang portion 200 using an adhesive to form dimples 201. Additionally, the thickness of the tang portion need not be increased by placing a dimple, as a portion of the tang portion may be folded over to increase the thickness. For example, in a tang portion manufactured from material having a thickness of about 0.1778 mm (0.007 inches), folding over the material would create a tang portion with a thickness of about 0.3556 mm (0.014 inches). Figs. 15A and 15B described below illustrate this concept as it relates to the axle hole of the jaw. The dimple 201 and/or tang portion 200 may be of any desired shape, size, dimensions, and/or configurations. For example, all the dimensions listed above are exemplary only.

In an exemplary embodiment of the invention, a tang portion of an end effector assembly of a medical device may have a widened and/or thickened portion, for example, by folding over material in a portion of the tang around the axle hole. As shown in Figs. 15A-15B, a tang portion 110 of an end effector, such as a jaw 114, may be formed such that it has an additional portion 111 extending from the tang portion 110. The additional portion 111 has through hole 113 with substantially the same diameter as an axle hole 112 of the rest of the tang portion 110. The additional portion 111 may then be folded over such that the through hole 113 is aligned with the axle hole 112. For example, a tang portion 110 may be stamped from a material having a thickness of about 0.1778 mm (0.007 inches). Thus, both the tang portion 110 and the additional portion 111 have a width of about 0.1778 mm (0.007 inches).
When folded over, the combined width of the tang portion 110 and the additional portion 111 becomes about 0.3556 mm (0.014 inches). A wider tang portion 110, and particularly a longer axle hole (the combined holes 112 and 113), may be advantageous because it imparts a wider footprint to the jaw mechanism, which may increase the stability and/or precision of the jaw, for example, during the clamping of opposing jaws.

In various embodiments, the tang portion may be widened by forming and then folding over multiple additional portions, for example, three additional portions. The width and/or thickness of other portions of a medical device, including other portions of the end effectors and/or end effector assembly, may be increased using this method. The folded over portion and/or tang portion may be of any desired shape, size, dimensions, and/or configurations. For example, all the dimensions listed above are exemplary only.

In another embodiment a tang portion of an end effector assembly of a medical device may have a portion configured to substantially prevent contact between an edge of the end effector and, for example, a tube-like member (such as an endoscope channel) in which the end effector assembly is configured to extend through or other external object. For example, in endoscopic applications, the jaws of a biopsy forceps device will follow the curvature of the endoscope. As the jaws pivot within an endoscope channel, the proximal tang behind the pivot may contact the channel wall. Biopsy jaws, including stamped biopsy jaws, may include sharp edges that may damage the endoscope channel.

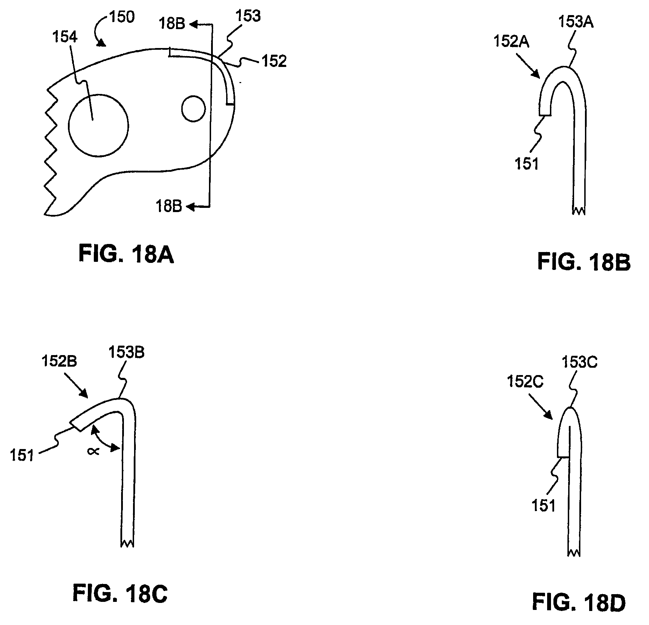

In an exemplary embodiment, as shown in Figs. 18A-18D, a portion 152 of the tang 150 may be folded over so as to substantially prevent an edge 151 of the tang 150 from contacting the inside of an endoscope channel. Instead, a smooth folded portion of the tang having a greater area will contact the endoscope channel. The portion 152 may be disposed on a proximal portion of the tang, however, the portion may also be disposed on any other suitable portion of the end effector. As shown in Fig. 18B, the portion 152A may be curved, however, the portion 152B may also be more sharply folded over as shown in Fig. 18C, or substantially completely folded over as shown in Fig. 18D (i.e., a portion of the folded over portion 152C substantially contacts another portion of the tang 150) so that the portion 152C may be substantially parallel with the tang 150.

The tang 150 may have an outer periphery 153 along its entire circumference. At an apex between the portion 152 and the rest of the tang 150, the outer periphery 153 may be the portion of the tang 150 that comes into contact with the inside of the endoscope channel, for example, as the end effector pivots about a pivot hole 154 of the tang 150. The outer periphery 153A, 153B, 153C at that apex is shown on the respective Figs. 18B-18D, and preferably has a smooth surface.

In various embodiments, the folded over portion may be folded on any side of the tang and/or may have any desired geometric configuration. For example, the folded over portion may form any desired angle α (see Fig. 18C) with the tang, e.g., more than 90 degrees, less than 90 degrees, and/or substantially 0 degrees. Manufacturing a folded over portion with an angle of more than 90 degrees relative to the remainder of the tang may be easier than manufacturing a folded over portion an angle of less than 90 degrees. However, a folded over portion with a less than a 90 degree angle to the tang may be more effective in substantially preventing contact between a sharp edge of the end effector and the endoscope channel. In examples, the folded over portion may have a substantially rounded shape (e.g., having a constant radius or a variable radius), for example, to present a smooth, non-damaging contact between the tang and the endoscope channel. The folded over portion may have a semi-circular shape of more than 180 degrees, less than 180 degrees, or substantially equal to 180 degrees. In a further example, the tang may have multiple portions configured to substantially prevent contact between an edge of the end effector and the endoscope channel.

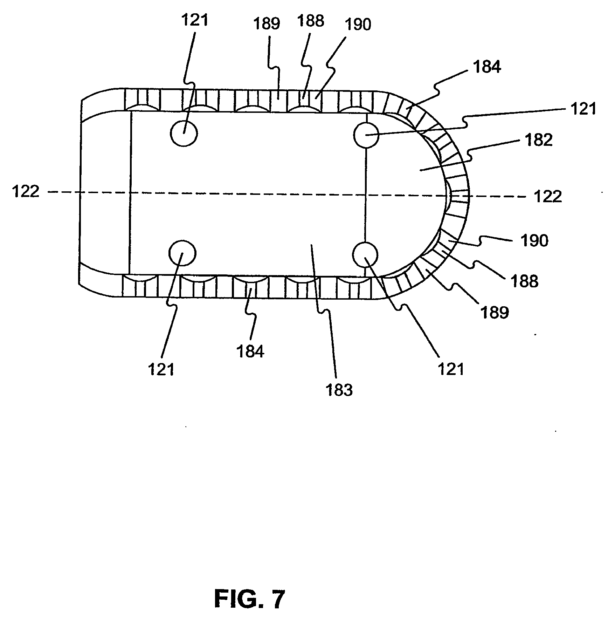

Embodiments include a medical device with holes in various portions of the medical device, including through the end effectors. For example, as shown in Fig. 7, a jaw 82 of a jaw assembly may have fenestration holes 121 in different portions of jaw 82. Fenestration holes 121 may assist in removing biopsy samples from the jaw 82, for example, by allowing fluid to enter the jaw 82 through the fenestration holes 121, flow between the biopsy sample and the jaw 82, and thus allow the biopsy sample to be flushed out of the jaw 82. The fenestration holes 121 may be disposed off a centerline 122 of the jaw 82. This may be advantageous as when the jaw 82 is placed down a channel, for example the working channel of an endoscope, because the jaw 82 may contact the inner wall of the channel substantially along its centerline 122, the channel will not come into contact with the fenestration holes 121. This may be desirable, for example, because contact between the holes 121 and the channel may cause the holes 121 to catch portions of the channel. This may cause damage to the channel and/or prevent the movement of the medical device with respect to the channel.

In various embodiments, the holes 121 may have any shape, for example, round, circular, oblong, square, and triangular. The holes 121 may also have of any size and have any desired dimensions. There may be any number of holes 121 on any portion of the medical device, but what is disclosed here are holes 121 that are not substantially located on the centerline 122 of the medical device and/or portions of the medical device that may come into contact with a channel and/or another object external to the medical device. The holes 121 need not necessarily be on portions of the medical device that completely preclude the holes 121 from coming into contact with the channel and/or another object external to the medical device, but may be on a portion where such contact is reduced or minimal relative to other portions of the medical device.

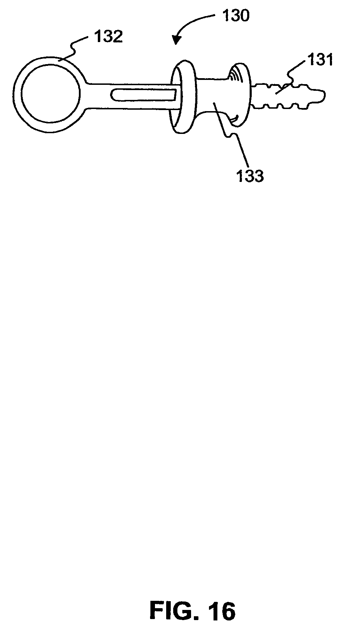

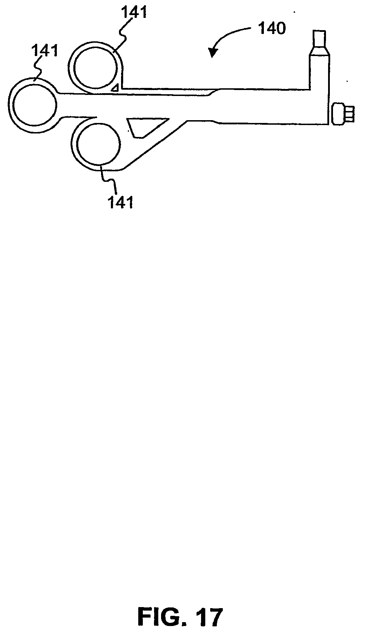

Embodiments include a medical device with user-interface portions configured to reduce stress (i.e. force) on the operator. For example, the handle of a medical device (e.g., an endoscopic instrument with a handle portion) may have soft-grip features. The entire handle may comprise the soft-grip features, or portions of the handle may have soft grip features, for example, those portions that accommodate a user's fingers. For example, in a handle 130 comprising a ring portion 132, an elongate portion 131, and a spool portion 133 disposed around the elongate portion 131, as shown in Fig. 16, the soft-grip features may be incorporated into the ring portion 132 and/or the spool portion 133. In another example, in a handle 140 comprising three-rings 141, as shown in Fig. 17, the soft-grip features may be incorporated into one or more of the three rings 141. The soft-grip feature may be a low durometer material, for example, santoprene or urethane, incorporated into the medical device. The soft-grip features reduce stress on the operator, for example, by reducing the stress on their hands, and have a more comfortable ergonomic feel. The reduction in stress on the user may allow the user to perform more procedures than with a medical device without the soft-grip features.

In various embodiments, any soft material may be used as soft-grip features, for example, rubber and/or rubbery thermoplastics. The soft-grip features may be placed on any portion of the medical device, for example, that have the potential to be handled by a user, provided that it does not otherwise interfere with the operation of the medical device. The soft-grip features may also be varied across portions of the device. For example, portions of the device may have different materials with different durometers.

Embodiments include a medical device having portions with variable stiffness. For example, in endoscopic instruments with an elongate member, portions of the elongate member may have variable stiffness. Some portions of the elongate member may be more flexible, for example, to allow the elongate member to be navigated through areas of the body having curves (i.e., tubular regions with greater tortuosity). Because of the flexibility, at least these portions of the elongate member may easily bend around even sharp curves, for example, in the gastrointestinal tract. Other portions of the elongate member may be more rigid, for example, to allow the elongate member to be properly advanced through areas of the body (e.g., tubular regions). Because of the rigidity, at least these portions of the elongate member can be pushed through, for example, the gastrointestinal tract.

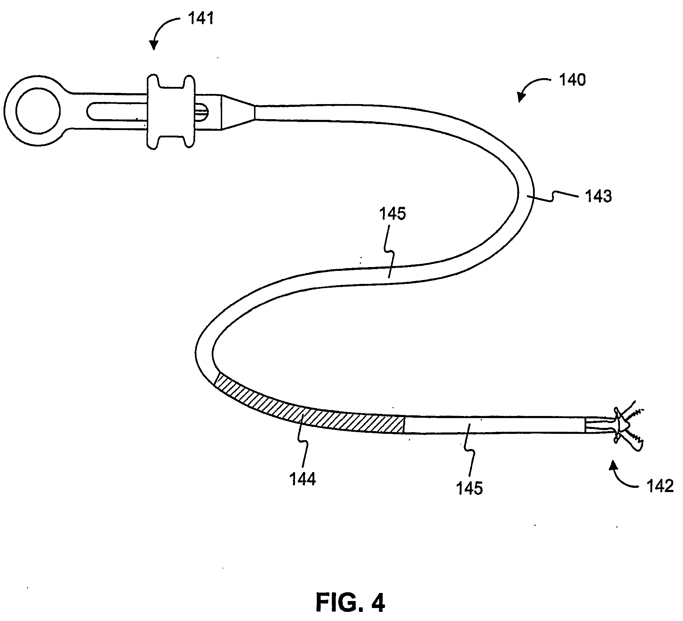

In an exemplary embodiment, Fig. 4 shows an endoscopic instrument 140 with a handle 141 and an end effector assembly 142 connected by an elongate member 143. The elongate member 143 may have a diameter of about 2.4 mm and a length of about 350 cm. However, any other dimensions suitable for its intended use are also possible. The entire elongate member 143 has a constant strength and feel from its proximal end to distal end, however, a portion 144 of the distal third of the elongate member 143 proximal to the distal end effector assembly has a lower stiffness than the other portions 145 of the elongate member 143. Methods of reducing the stiffness of the desired portion 144 of the elongate member 143 include reducing the diameter of the elongate member 143 in the targeted area, and/or varying the material used in the elongate member 143 such that the lower stiffness portion 144 is comprised of a more flexible material than the higher stiffness portions 145.

In various embodiments, the elongate member may have its rigidity varied along any portion of the elongate member, may have multiple portions with multiple levels of stiffness, and/or may be manufactured using any method known in the art.

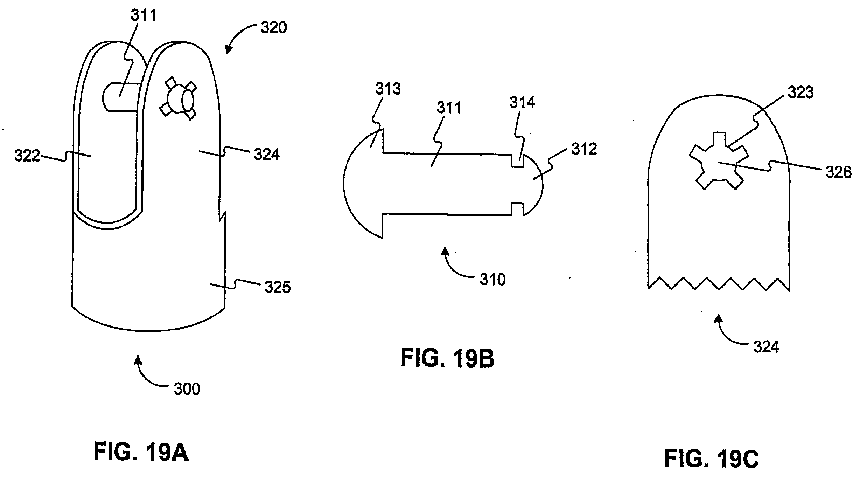

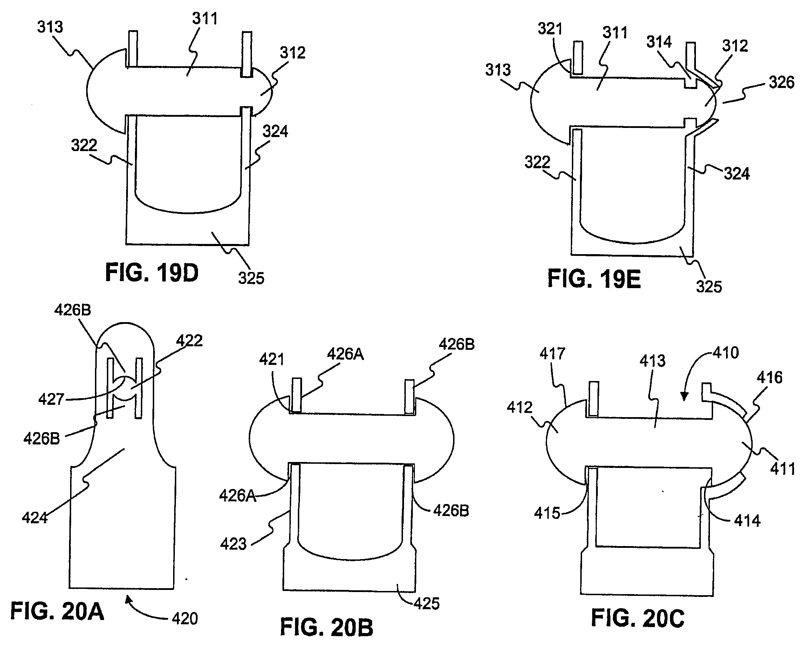

Embodiments include a clevis assembly. An exemplary embodiment of a clevis assembly 300 is shown in Figs. 19A-19E. The clevis assembly 300 may include an axle 310 and a clevis 320.

The axle 310 may be generally elongate in shape and configured to be used with clevis 320. The axle 310 may have a central portion 311 disposed between ends 312, 313. The central portion 311 may be substantially cylindrical in shape and may be configured to be placed through a hole 321 on one of the arms 322 of the clevis 320. The central portion 311 may also be configured to accommodate a portion of an end effector assembly, such as the proximal tang portions of biopsy jaws.

One end 313 of the axle 310 may be configured to prevent the end 313 from being placed through the hole 321 on the clevis arm 322. The end 313 may include an enlarged head with a shoulder. The head may be substantially hemispherical in shape, however, the end 313 may also have any suitable shape or configuration to prevent its extension through the hole 321.

The end 312 may be substantially round in shape, and may have a groove 314 that separates the end 312 from the central portion 311. The groove 314 may extend all the way around the axle 310, and may be configured to receive a portion of one or more of the protrusions, or cantilever arms, 323 extending around a hole 326 defined by another clevis arm 324.

The clevis 320 may have a base 325 from which arms 322, 324 extend. The arms 322, 324 may be substantially similar in shape, however, they may also have different shapes or configurations. One arm 322 has hole 321 configured to accommodate a portion of axle 310, for example, the central portion 311 of axle 310. Another arm 324 has a hole 326 with a non-uniform edge 327 that is defined by one or more protrusions 323. The protrusions 323 may each have substantially the same shape, or may have different shapes and/or configurations (e.g., spacing). The holes 321, 326 may be substantially coaxial. The portion of the arm 324 defining the hole 326 may be configured to bend or deflect as axle 310 is placed through the hole 326. For example, as shown in Fig. 19E, the protrusions 323 may deflect away from the arm 322 as end 312 of the axle 310 is placed through the hole 326. The portion of the arm 324 defining the hole 326 may also be configured to substantially return to its original configuration. For example, once the end 312 of the axle 310 has been placed through the hole 326 a suitable amount, the protrusions 323 may deflect or spring back toward the arm 322 and at least a portion of the protrusions 323 may become lodged in groove 314.

The portion of the arm 324 not defining the hole 326 and/or protrusions 323 may be configured to be rigid enough such that the arm 324 does not substantially bend or deflect while the protrusions 323 bend or deflect as the end 312 of the axle 310 is placed through the hole 326. For example, the portion of the arm 324 not defining the hole 326 may be thicker than the protrusions 323. The base 325 may also be configured to be more rigid than the arms 322, 324, for example, so as to not substantially bend or deflect while the protrusions 323 may bend or deflect as the end 312 of the axle 310 is placed through the hole 326. In another example, the portion of the arm 324 not defining the hole 326 and/or protrusions 323 may not have any particular configuration or rigidity such that the arm 324 does not substantially bend or deflect while the protrusions 323 bend or deflect as the end 312 of the axle 310 is placed through the hole 326. For example, arm 324 may simply have roughly the same thickness, rigidity, and/or metallic properties as the rest of the clevis assembly 300. In such cases, tooling may be used to prevent deflection of the arm 324. For example, the arm 324 may be placed between grippers, vices, or any other suitable tooling known in the art so as to substantially prevent deflection of the arm 324 in a direction substantially perpendicular to the surface of the arm 324 and/or substantially parallel to the longitudinal axis of the axle 311 (e.g., when the end 312 of the axle 310 is placed through the hole 326 and exerts force on the protrusions 323).

Another exemplary embodiment of a clevis assembly 400 is shown in Figs. 20A-20C. The clevis assembly 400 may include an axle 410 and a clevis 420.

The axle 410 may have two ends 411, 412 disposed around a central portion 413. The central portion 413 may be substantially cylindrical in shape and may be configured to be disposed in holes 421, 422 on arms 423, 424 on the clevis 420. The central portion 413 may also be configured to accommodate a portion of an end effector assembly, such as the proximal tang portions of biopsy jaws.

The ends 411, 412 may have a generally rounded shape and may be configured to prevent the ends 411, 412 from being placed through at least one of the holes 421. For example, the ends 411, 412 may include an enlarged head and a shoulder. The head may be substantially hemispherical in shape, however, the ends 411, 412 may have any suitable shape or configuration. An inner surface 414, 415 of the ends 411, 412 may be configured to prevent the rest of the end 411, 412 from being placed through holes 421, 422. An outer surface 416, 417, however, may be configured to be placed through at least one of the holes 421, 422. The ends 411, 412 may have substantially the same shape and configuration, or may have different shapes and/or configurations. For example, one of the ends 411, 412 may be configured so that it may not be placed through at least one of the holes 421, 422.

The clevis 420 may have a base 425 from which arms 423, 424 extend. The arms 423, 424 may have substantially similar shapes, or may have different shapes and/or configurations. One or more of the arms 423, 424 may define a hole 421, 422 with one or more protrusions 426A, 426B adjacent portions of the hole 421, 422. The protrusions 426A, 426B may have the same shape, or may have different shapes. The protrusions 426A, 426B may define substantially rounded inner edges 427 that are configured, for example, to define portions of a circle. The protrusions 426A may be configured to deflect toward arm 424 as end 411 is placed through the hole 421. The protrusions 426B may be configured to deflect away from arm 423 as end 411 is placed through the hole 422. As shown in Fig. 20C, when an outer surface 416 of an end 411 of an axle 410 is pressed against the protrusions 426B, the protrusions 426B may deflect as the end 411 is advanced through hole 422. Once the end 411 has suitably advanced through the hole 422, the protrusions 426B may reversibly deflect toward the arm 423 such that the inner edges 427 are adjacent an outer surface of the central portion 413. In such a configuration, the inner surfaces 414, 415 of the ends 411,412 may be adjacent outer surfaces of the arms 423, 424. The same may substantially be true for hole 421 and protrusions 426A, except that the outer surface 416 of the end 411 of the axle 410 may first come into contact with an outer surface of arm 423, and the protrusions 426A may deflect inward (i.e., toward arm 424).

The portion of the arm 423, 424 not defining the hole 421, 422 and/or protrusions 426A, 426B may be configured to be rigid enough such that the arm 423, 424 does not substantially bend or deflect while the protrusions 426A, 426B may bend or deflect as the end 411 of the axle 410 is placed through the hole 421,422. For example, the portion of the arm 423, 424 not defining the hole 421, 422 may be thicker than the protrusions 426A, 426B. The base 425 may also be configured to be more rigid than the arms 423, 424, for example, so as to not substantially bend or deflect while protrusions 426A, 426B may bend or deflect as the end 411 of the axle 410 is placed through the hole 421, 422. In another example, the portion of the arm 423, 424 not defining the hole 421, 422 and/or protrusions 426A, 426B may not have any particular configuration or rigidity such that the arm 423, 424 does not substantially bend or deflect while the protrusions 426A, 426B bend or deflect as the end 411 of the axle 410 is placed through the hole 426A, 426B. For example, arms 423, 424 may have roughly the same thickness, rigidity, and/or metallic properties as the rest of the clevis assembly 420. In such a case, tooling may be used to prevent deflection of the arm 423, 424. For example, the arm 423, 424 may be placed between grippers, vices, or any other suitable tooling known in the art so as to substantially prevent deflection of the arm 423, 424 in a direction substantially perpendicular to the surface of the arm 423 424 and/or substantially parallel to the longitudinal axis of the axle 410 (e.g., when the end 411 of the axle 410 is placed through the hole 421, 422 and exerts force on the protrusions 426A, 426B).

In various embodiments, each arm of the clevis may define a hole with protrusions configured to deflect and then return to its original configuration as an axle is placed therethrough, substantially as set forth above. However, in other embodiments, clevis arms may have different configurations. For example, one of the arms may define a hole with protrusions configured to deflect and then return to its original configuration as an axle is placed therethrough, however, the other arm may define a hole without protrusions that is otherwise configured to allow an end of an axle to pass through the hole without substantially deflecting any portion of the arm. In such a configuration, one end of the axle may have a small enough size and/or shape to pass through the hole on one of the arms and then deflect the protrusions adjacent the hole on the other arm as the end passes therethrough.

There may be several advantages to having a clevis assembly with an axle and clevis configuration according to one of the embodiments set forth herein, for example, Figs. 19A-19E and 20A-20C. One advantage is the elimination of a rivet and the use of expensive riveting equipment to manufacture the clevis assembly. Another advantage is that the clevis assembly may be assembled quickly and through an automated process. A further advantage is that the axle may be solid and thus less expensive than hollow axles which may be used in other clevis assembly configurations. Yet another advantage is that the axle may not include sharp points or edges that may damage the walls of a working channel of an endoscope through which the clevis assembly may be placed. Still another advantage is that the groove may be accurately and precisely placed on the axle such that when the clevis assembly is assembled and the protrusions on the hole of one of the arms are disposed in the groove, the resulting distance between the arms may be precisely controlled and/or ideally manufactured for the intended use of the clevis assembly.

Other embodiments of the invention will be apparent to those skilled in the art from consideration of the specification and practice of the invention, which is defined the following claims.

## Claims

1. A medical device comprising:
a handle (11);
an end effector assembly (12); and
a member (13) connecting the handle (11) to the end effector assembly (12),
wherein the end effector assembly (12) comprises an end effector (114) with a distal end effector portion and a proximal tang portion (110), **characterized by** the tang portion (110) having an additional portion (111) extending from the tang portion (110), the additional portion (111) having a through hole (113) with the same diameter as an axle hole (112) of the tang portion (110), and the additional portion (111) being folded over such that the through hole (113) is aligned with the axle hole (112)

2. The medical device of claim 1, further including a wire (101), wherein the tang portion (110) further defines a mounting hole (102) configured to receive a wire (101), and
wherein the wire (101) has a first wire portion contacting one side of the tang portion (110) and a second wire portion contacting another side of the tang portion (110).

3. The medical device of claim 2, wherein the wire (101) is bent on both sides of the mounting hole (102).

4. The medical device of claim 1, wherein the end effector assembly (12) includes two end effectors (114).

## Patentansprüche

1. Medizinische Vorrichtung mit:
einem Griff (11);
einer Endeffektoranordnung (12); und
einem Element (13), das den Griff (11) mit der Endeffektoranordnung (12) verbindet,
wobei die Endeffektoranordnung (12) einen Endeffektor (114) mit einem distalen Endeffektorabschnitt und einem proximalen Montageabschnitt (110) aufweist,
**dadurch gekennzeichnet, dass** der Montageabschnitt (110) einen zusätzlichen Abschnitt (111) aufweist, der sich von dem Montageabschnitt (110) erstreckt, wobei der zusätzliche Abschnitt (111) ein Durchgangsloch (113) aufweist, das einen Durchmesser hat, der gleich dem Durchmesser eines Achsenlochs (112) des Montageabschnitts (110) ist, und wobei der zusätzliche Abschnitt (111) gefaltet ist, so dass das Durchgangsloch (113) mit dem Achsenloch (112) ausgerichtet ist.

2. Medizinische Vorrichtung nach Anspruch 1, ferner mit einem Draht (101), wobei der Montageabschnitt (110) ferner ein Montageloch (102) begrenzt, das konfiguriert ist, einen Draht (101) aufzunehmen, und
wobei der Draht (101) einen eine Seite des Montageabschnitts (110) kontaktierenden ersten Drahtabschnitt und einen eine andere Seite des Montageabschnitts (110) kontaktierenden zweiten Drahtabschnitt aufweist.

3. Medizinische Vorrichtung nach Anspruch 2, wobei der Draht (101) an beiden Seiten des Montagelochs (102) gebogen ist.

4. Medizinische Vorrichtung nach Anspruch 1, wobei die Endeffektoranordnung (12) zwei Endeffektoren (114) aufweist.

## Revendications

1. Dispositif médical comprenant :
une manette (11) ;
un ensemble d'effecteur d'extrémité (12) ; et
un élément (13) raccordant la manette (11) à l'ensemble d'effecteur d'extrémité (12),
dans lequel l'ensemble d'effecteur d'extrémité (12) comprend un effecteur d'extrémité (114) avec une partie d'effecteur d'extrémité distale et une partie de fût proximale (110), **caractérisé par** la partie de fût (110) qui a une partie supplémentaire (111) s'étendant à partir de la partie de fût (110), la partie supplémentaire (111) ayant un trou débouchant (113) avec le même diamètre qu'un trou d'axe (112) de la partie de fût (110), et la partie supplémentaire (111) étant repliée de sorte que le trou débouchant (113) est aligné avec le trou d'axe (112).

2. Dispositif médical selon la revendication 1, comprenant en outre un fil (101), dans lequel la partie de fût (110) définit en outre un trou de montage (102) configuré pour recevoir un fil (101), et
dans lequel le fil (101) a une première partie de fil en contact avec un côté de la partie de fût (110) et une seconde partie de fil en contact avec un autre côté de la partie de fût (110).

3. Dispositif médical selon la revendication 2, dans lequel le fil (101) est plié des deux côtés du trou de montage (102).

4. Dispositif médical selon la revendication 1, dans lequel l'ensemble d'effecteur d'extrémité (12) comprend deux effecteurs d'extrémité (114).
